# EUROPEAN PATENT APPLICATION

(11) **EP 2 233 134 A1**
(43) Date of publication of application: **29.09.2010**
(21) Application number: 10250622.7
(22) Date of filing: 26.03.2010
(51) Int. Cl.: A61K 9/20, A61K 9/00

(54) **Multi-portion intra-oral dosage form with organoleptic properties**

(30) Priority: 27.03.2009 SE 0900397; 27.04.2009 US 430238
(71) Applicant: McNeil AB, 25109 Helsingborg (SE)
(72) Inventor: Hugerth, Andreas, 237 33 Bjärred (SE); Lindell, Katarina, 241 93 Eslöv (SE); Thyresson, Kristina, 226 57 Lund (SE); Nicklasson, Fredrik, 237 31 Bjärred (SE)
(74) Representative: Kirsch, Susan Edith

(57) **Abstract**

The present invention relates to a multi portion intra-oral dosage form comprising at least one pharmaceutically active agent or health promoting agent wherein at least one portion comprises a component for creating a noticeable organoleptic sensation.

Of certain interest is use of sensory markers/signals as conceptual aids for a subject using the dosage form whereby the organoleptic sensation/s is/are such that it/they facilitate/s for the subject to identify a portion and differentiate between different portions thereof. Also contemplated are a dosage form, a method and a system for delivering active agents, such as nicotine and/or metabolites thereof, such as cotinine, nicotine N'-oxide, nornicotine, (S)-nicotine-N-β-glucuronide and mixtures, isomers, salts and complexes thereof as well as use and production of said dosage forms.

## Description

### Field of the Invention

The present invention relates to a multi portion intra-oral dosage form comprising at least one pharmaceutically active or health promoting agent, wherein at least one portion comprises a component for creating a noticeable organoleptic sensation.

Of certain interest is use of sensory markers/signals as conceptual aids for a subject using the dosage form whereby the organoleptic sensation/s is/are such that it/they facilitate/s for the subject to identify different portions and differentiate between the different portions thereof. Also contemplated are a method and a system for delivering active agents, such as nicotine and/or metabolites thereof, such as cotinine, nicotine N'-oxide, nornicotine, (S)-nicotine-N-β-glucuronide and mixtures, isomers, salts and complexes thereof as well as use and production of said dosage forms.

### Background of the Invention

Tobacco dependence and reduction thereof is a desirable goal. In recent years, with the recognition of the harmful effects of tobacco smoking, there have been numerous campaigns and programs by governmental agencies and various health groups and other interested organisations to disseminate information about the adverse health effects resulting from tobacco smoking. Moreover, and as a result of this recognition of the harmful effects, there have been many programs directed to attempts in reducing smoking incidence.

Nicotine is an organic compound and is the principal alkaloid of tobacco. Nicotine is the chief addictive ingredient in the tobacco used in cigarettes, cigars, snuff and the like. Nicotine is also an addictive drug, and smokers characteristically display a strong tendency to relapse after having successfully stopped smoking for a time. Nicotine is the world's second most used drug, after caffeine from coffee and tea.

The main problem with tobacco smoking is its enormous implications on health. It is estimated that smoking related diseases cause some 3 - 4 million deaths per year. According to Centers for Disease Control and Prevention, cigarette smoking among adults - United States, 1995. MMWR 1997; 46:1217 - 1220 around 500,000 persons in USA die each year as a result of tobacco use. In fact, excessive smoking is now recognised as one of the major health problems throughout the world. This grim consequence of tobacco smoking has urged many medical associations and health authorities to take very strong actions against the use of tobacco.

Even though tobacco smoking is decreasing in many developed countries today it is hard to see how the societies could get rid of the world's second most used drug. The incidence of smoking is still rising in many countries, especially in less developed countries.

The most advantageous thing a heavy smoker can do is to stop smoking completely or at least to reduce his/her smoking. Experience shows, however, that most smokers find this extremely difficult since, mostly, tobacco smoking results in a dependence disorder or craving. The World Health Organization ("WHO") has in its International Classification of Disorders a diagnosis called Tobacco Dependence. Others like the American Psychiatric Association call the addiction Nicotine Dependence. It is generally accepted that these difficulties to stop smoking result from the fact that those heavy smokers are dependent on nicotine. The most important risk factors related to health are, however, substances that are formed during the combustion of tobacco, such as carcinogenic tar products, carbon monoxide, N-nitrosamines, aldehydes, and hydrocyanic acid.

### Effects of nicotine

Nicotine is an addictive alkaloid C₅H₄NC₄H₇NCH₃, derived from the tobacco plant. Nicotine is also used as an insecticide. The administration of nicotine (for example, in the form of smoking a cigarette, cigar or pipe) can give a pleasurable feeling to the smoker. However, smoking has health hazards and it is, therefore, desirable to formulate an alternative way of administering nicotine in a pleasurable and harmless manner that can be used to facilitate withdrawal from smoking and/or used as a replacement for smoking.

When smoking a cigarette, nicotine is quickly absorbed into the smoker's blood and reaches the brain within around ten seconds after inhalation. The quick uptake of nicotine gives the consumer a rapid satisfaction, or kick. The satisfaction usually lasts during the smoking time of the cigarette and for a period of time thereafter. The poisonous, toxic, carcinogenic, and addictive nature of smoking has provided strong motivation to develop methods, compositions and devices, which can be used to break the habit of smoking cigarettes.

### Nicotine replacement products

One way to reduce smoking is to provide nicotine in a form or manner other than by smoking and some products have been developed to fulfil this need. Nicotine containing formulations are currently the dominating treatments for tobacco dependence. The successes in achieving reduction in the incidence of smoking have been relatively poor using presently known products. The present state of the art involves both behavioural approaches and pharmacological approaches. More than 80 % of the tobacco smokers who initially quit smoking after using some behavioural or pharmacological approach to singly reduce smoking incidence generally relapse and return to the habit of smoking at their former rate of smoking within about a one year's period of time.

As an aid for those who are willing to stop smoking there are several ways and forms of nicotine replacement products available on the market. Several methods and means have been described for diminishing the desire of a subject to use tobacco, which comprises the step of administering to the subject nicotine or a derivative thereof as described in e g U.S. Patent Number 5,810,018 (oral nicotine-containing spray), U.S. Patent Number 5,939,100 (nicotine-containing micro spheres) and U.S. Patent Number 4,967,773 (nicotine-containing lozenge).

Nicotine-containing nose drops have been reported (Russell et al., British Medical Journal, Vol. 286, p. 683 (1983); Jarvis et al., Brit. J. of Addiction, Vol. 82, p. 983 (1987)). Nose drops, however, are difficult to administer and are not convenient for use at work or in other public situations. Administrating nicotine by way of delivering directly into the nasal cavity by spraying is known from U.S. Patent Number 4,579,858, DE 32 41 437 and WO93/12764. There may be local nasal irritation, however, with use of nasal nicotine formulations. The difficulty in administration also results in unpredictability of the dose of nicotine administered.

The use of skin patches for transdermal administration of nicotine has been reported (Rose, in Pharmacologic Treatment of Tobacco Dependence, (1986) pp. 158-166, Harvard Univ. Press). Nicotine-containing skin patches that are in wide use today can cause local irritation and the absorption of nicotine is slow and affected by cutaneous blood flow.

Also, inhaling devices resembling a cigarette are known for uptake of nicotine vapours as suggested in U.S. Patent Number 5,167,242. Said means and methods address the problems associated with addiction to nicotine.

One successful product that is used as a smoking substitute and/or as a smoking cessation aid and which is based on nicotine is the chewing gum Nicorette^{®}. This product was one of the first nicotine replacement forms that was approved by the Food and Drug Administration (FDA) and is still one of the most used nicotine replacement products. Nicorette^{®} chewing gum has been on the market in about 60 countries for several years. In this chewing gum the nicotine is present in the form of a complex with an insoluble cation-exchanger (polacrilex) that is dispersed in a gum base. The nicotine is slowly released from the gum due to chewing and will reach similar plasma levels as when smoking a cigarette after about 30 minutes depending on the chewing technique, i e slow or active. Patents related to this product are e g U.S. Patent Number 3,877,468, U.S. Patent Number 3,901,248 and U.S. Patent Number 3,845,217.

Other successful nicotine replacement products are Nicorette® Microtab and its successor Nicorette® Microtab Lemon. These tablets are sublingual tablets and provide slow release of nicotine that aids a subject to achieve a nicotine plasma profile similar (bioequivalent) to that of the Nicorette® chewing gum.

Pharmaceuticals intended for oral administration are typically provided in solid form as tablets, capsules, pills, lozenges, or granules. Rapidly dissolving tablets are often employed in the administration of pharmaceuticals where it is impractical to provide a tablet for swallowing whole, for instance with paediatric patients. Several workers in the field have explored rapidly disintegrative tablets, e g U.S. Patent Nos. 6,106,861 and 6,024,981 and PCT Application No. WO 99/47126.

### Prior art

US 5,879,710 discloses a specific mucoadhesive double layer formulation for administration of melatonin.

US 5,236,713 discloses a laminated preparation for intermittently releasing an active agent.

WO 1992/01445 discloses an osmotic device for controlled delivery of nicotine base through an oral mucosa membrane.

US 20060073189A1 discloses monolayer oral preparations for biphasic delivery of nicotine.

US 5,681,583 discloses a double-layer tablet to be swallowed for administration of an active material, whereby one layer releases the active quickly, while the other layer releases the active more gradually. A tablet to be swallowed is intended for uptake of an active in the GI tract, which is totally different from a dosage form for intraoral uptake of an active.

US 200301 18648A1 discloses a pharmaceutical composition comprising a moulded triturate portion surrounded by a compressed annular tablet comprising a pharmaceutically active ingredient.

WO2001/037814 discloses a tablet that is attachable to the buccal mucosa, where it releases a substance in a multiphasic manner, typically with an initial burst release followed by controlled release over a longer period. '814 though does not comprise any proof of utility for this concept.

US 6,248,760 discloses a multi-layered nicotine-containing tablet where a non-toxic matrix layer comprises an antacid, but does not contain nicotine.

Anyhow, none of the above mentioned references sufficiently well addresses compliance and efficacy. Neither do they provide any solution to the problem on how to differentiate between different portions of a multiportion dosage form.

### Detailed Description of the Invention

### Definitions

The below definitions apply *mutatis mutandis* on expressions being similar to those being defined below.

The term "organoleptic sensation" is herein intended to mean a feature of the embodiments of the present invention that is discernable to the taste, mouth feel, smell, hearing and/or vision of the subject such as, but not limited to, flavor, cooling, burning, warming, heating, tingling, crunchiness, crumbliness, flakiness, fusing, mouth watering, color, size, shape, auditive effect, effervescence, visual effect, stickiness, fragrance, olfactory sensation, bubbling, foaming, viscosity, elasticity, rheology, texture, e g hardness, softness, mouth feel, smoothness, roughness, embossing and engravings, and difference in dissolution rate. Preferably, the organoleptic sensation/s is/are related to perception of flavor, cooling, burning, warming, heating, tingling, crunchiness, crumbliness, flakiness, fusing, mouth watering, color, size, shape, auditive effect, effervescence, visual effect, stickiness, fragrance, olfactory sensation, bubbling, foaming, viscosity, elasticity, rheology, texture, e g hardness, softness, mouth feel, smoothness, roughness, embossing and engravings, and difference in dissolution rate, most preferably the organoleptic sensation/s is/are related to perception of flavor and/or difference in dissolution rate.

In addition "organoleptic sensation" can also be a feature resulting from the absence of an "organoleptic sensation" discernable in a different portion. Organoleptic sensations may for example be obtained as follows, cooling through use of cooling agent in one or several portion and no cooling agent added in other portion/s, difference in dissolution rate between one or several portions through inter-portion composition differences and/or inter-portion production differences, texture and/or shape through use of inter-portion composition differences and/or inter-portion production differences resulting in inter-portion differences such as, but not limited to, geometric shape/form, hardness, softness, mouth feel, flakiness, stickiness, crunchiness, smoothness, roughness and engravings, burning through use of burning agent/s, where the pharmaceutically active agent may also be a provider of a/the burning sensation/s in one portion or several portions and no burning agent added in other portion/s, mouth watering through use of a mouth watering agent/s in one portion or several portions and no added watering agent/s in other portion/s, warming/heating through use of warming agent/s, where the pharmaceutically active agent may also be provider of a/the warming/heating sensation/s in one portion or several portions and no warming agent added in other portion/s and tingling through use of tingling agent/s in one portion or several portions and no tingling agent/s added in other portion/s, and any combination/s thereof or with any other organoleptic sensation.

The term "nicotine mimicking component" is herein intended to mean a component that in some respects may be considered to share or resemble any organoleptic feature of nicotine irrespective of the form of nicotine

The terms "intra-oral dosage form" and "oral dosage form" is herein intended to mean a dosage form intended for administration into the systemic blood circulation by means of absorption of an active principle, i e a pharmaceutically active compound, by any tissue of the oral cavity.

The term "complete reduction" is herein intended to mean complete or substantially complete reduction.

The term "controlled release" is intended to mean a release of a pharmaceutical or health-promoting agent from an oral formulation in the oral cavity of the subject, whereby active sucking or other manipulation of the oral formulation is controlling the amount of the agent being released.

The term "portion" is intended to mean a separate entity of a dosage form. Examples of a portion are e g a tablet layer, a hard boiled candy layer, a melt layer, a capsule, a coating, a wine gum, and a chewing gum.

The term "disintegration" is intended to mean loss of integrity of a portion through crumbling, dissolution, melting or other dispersion of the contents of the portion.

The term "dissolution" is intended to mean disintegration of a portion in such a way that subsequent solubilization gives rise to a molecular dispersion (i.e. a solution) of the contents of the portion.

The term "slow release" is intended to mean that a pharmaceutically active agent, e g nicotine, is released from the oral formulation upon sucking or other manipulation over a period of time, for example several minutes to an hour.

The term "unit formula" is intended to mean one multi portion intra-oral formulation unit.

The term "transient" is intended to mean a non-permanent change, upon which the relevant state, e g biological or physiological state, after a certain period of time will return to its value or behaviour prior to said change.

The terms "buccal" and "buccally" are herein intended to pertain to all of or any part of the tissue of the oral cavity.

The term "compressible excipient" is here intended to mean an ingredient that can be compressed into a dosage form without the addition of any further binding agents.

The term "water swellable excipient" is here intended to mean a material that is designed to swell or wick liquid upon contact with a liquid medium and to aid in the dissolution of the compressed tablet.

The term "health promoting agent" is here intended to mean any agent that may be envisaged to have a beneficial effect, direct or indirect, on the health and/or wellbeing of a subject and may include but are not limited to agents such as a tooth whitening agent, a breath freshening agent, an oral health promoting agent, an anti-caries agent, salivation increasing agent(s) and herbal extract(s).

### Summary of the Invention

There is a long felt and unmet need for improved compliance and efficacy for most pharmaceutically active and/or health promoting agents intended for oral and/or per oral administration. The present invention relates to a multi portion intra-oral dosage form comprising at least one pharmaceutically active or health promoting agent, wherein at least one portion comprises a component for creating a noticeable organoleptic sensation, such that there is a discernable difference un organoleptic sensation between at least two portions.

Preferably, the portions of the multi portion intra-oral dosage form have different disintegration rates.

The present invention further relates to the use of a multi-portion intra-oral dosage form according to the invention comprising nicotine in any form for obtaining a quick and/or sustained and/or complete reduction of the urge to smoke or use of tobacco and/or for providing a sense of smoking satisfaction without smoking and/or withdrawal symptom relief and/or for creating a nicotine kick.

The present invention further relates to a multi-portion intra-oral dosage form according to the inventioncomprising nicotine in any form for use in therapy wherein the therapy is treatment of a disease selected from the group consisting of tobacco or nicotine dependence, Alzheimer's disease, Crohn's disease, Parkinson's disease, Tourette's syndrome, ulcerous colitis and post-smoking-cessation weight control.

The dosage form may e g be a lozenge, a tablet, an oral film, a chewing gum, a sublingual tablet, a troche, a lolly pop, a hard boiled candy, a chocolate lens, a micro bead, a wine gum, a semi solid, or a combination thereof.

Of certain interest is the use of sensory markers/signals as conceptual aids for a subject using the dosage form, whereby the organoleptic sensation/s is/are such that it/they facilitate/s for the subject to differentiate between different portions thereof. It is envisaged that by providing conceptual aids the association of a certain organoleptic sensation with a certain functionality of the potential embodiments of the invention provides means for increased awareness of the functionality and constituents of the dosage form and hence increase the patient's compliance to the medication as well as provide means for improving current therapy and efficacy by combining phamaceutically active agents and/or health promoting agents in novel and more advantageous ways.

For example, the multi portion intra-oral dosage form according to the invention may deliver an organoleptic sensation from a portion as a signal to inform a subject using the dosage form that the pharmaceutically active agent or health promoting agent being present in said portion has started to be released there from.

The multi portion intra-oral dosage form according to the invention may provide the organoleptic sensation from a portion as a signal to inform a subject using the dosage form that all of or a fraction of a pharmaceutically active agent or health promoting agent being present in said portion has been released there from.

Nicotine in any form and/or a nicotine-mimicking compound may be included in one or several portions of the dosage form.

An object of the present invention is thus to provide an efficient and effective intra-oral dosage form, as well as methods and systems for delivering e g nicotine and/or metabolites thereof, such as cotinine, nicotine N'-oxide, nornicotine, (S)-nicotine-N-β-glucuronide and mixtures, isomers, salts and complexes thereof and/or a nicotine-mimicking compound and optionally component/components for creating an organoleptic sensation to a subject so as to obtain a transmucosal uptake of nicotine and/or metabolites thereof and mixtures, isomers, salts and complexes thereof in the oral cavity of the subject, as well as a method for producing said intra-oral dosage form.

A suitable method for obtaining reduction of the urge to smoke or use tobacco containing material and/or for providing a sense of smoking satisfaction without smoking, comprises the steps of replacing at least partly the tobacco containing material with the multi portion intra-oral dosage form, administering to a subject the multi portion intra-oral dosage form containing nicotine and/or metabolites thereof, and mixtures, isomers, salts and complexes thereof in any form into the oral cavity of the subject and if needed allowing the nicotine and/or metabolites thereof, such as cotinine, nicotine N'-oxide, nornicotine, (S)-nicotine-N-β-glucuronide and mixtures, isomers, salts and complexes thereof in any form of the oral formulation to be released in the saliva in the oral cavity and absorbed into the systemic circulation of the subject.

A suitable system for delivering nicotine and/or metabolites thereof, such as cotinine, nicotine N'-oxide, nornicotine, (S)-nicotine-N-β-glucuronide and mixtures, isomers, salts and complexes thereof in any form to a subject, comprises said intra-oral dosage form and at least one other means for obtaining reduction of the urge to smoke or use of tobacco. A suitable system for obtaining reduction of the urge to smoke or otherwise use tobacco and/or for providing a sense of smoking satisfaction without smoking, comprises an multi portion intra-oral dosage form of the invention and at least one other method for obtaining reduction of the urge to smoke or otherwise use tobacco. Said systems may be systems wherein the at least other method is selected from the group consisting of administration through, nasal sprays, transdermal patches, inhaling devices, lozenges, tablets, chewing gum and parenteral methods, subcutaneous methods, transmucousal methods; or other use of tobacco; and/or behavioural therapy. Preferably the at least other means or method comprises administration of nicotine.

In addition, the present invention may also be used for the production of an intra-oral dosage form comprising nicotine and/or metabolites thereof, such as cotinine, nicotine N'-oxide, nornicotine, (S)-nicotine-N-β-glucuronide and mixtures, isomers, salts and complexes thereof in any form for use in therapy wherein the therapy is treatment of a disease selected from the group consisting of tobacco or nicotine dependence, Alzheimer's disease, Crohn's disease, Parkinson's disease, Tourette's syndrome, ulcerous colitis and post-smoking-cessation weight control.

Other features and advantages of the present invention will be apparent from the detailed description of the invention and from the claims.

### Pharmaceutically Active and Health Promoting Agents

The present invention may thus be employed in embodiments where for example, but not limited to, one or more pharmaceutically active and/or health promoting agent(s) is/are chosen from anti-inflammatory agents, for example diclofenac, ketorolac, indometacin, tornoxicam, piroxicam, tenoxicam, ketoprofen, celecoxib and roficoxib; muscle relaxants, for example orphenadrine and baclofen; drugs affecting bone mineralization, for example alendronic acid and risedronic acid; analgesics, for example propoxyphene, buprenorfin, ketobenidon, hydromorphone, tramadol, morphine, and tapentadol; antimigraine preparations, for example dihydroergotamine, ergotamine, eletriptan, naratriptan, rizatriptan, sumatriptan and zolmitriptan; anti-Parkinson, drugs for example levodopamine, carbidopamine, pramipexole, ropinirole and selegiline; anxiolytics, for example alprazolam, diazepam, lorazepam and oxazepam; hypnotics, for example flunitrazepam, midazolam, nitrazepam, triazolam, zaleplone, zopiclone, zolpiderm, clometiazole and propiomazine; psychostimulant, for example caffeine; drugs against substance dependence for example bupropione, lobeline, naltrexone and methadone; gastric ulcer remedy for example famotidine; antispasmodic, for example hyoscyamine; antiemetics, for example metoclopramide, ondansetron, scopolamine, hyoscine, perfenazine, procloperazine and haloperidol; antidiabetic agents, for example rosiglitazone as well as other glitazones; cardiovascular agents, for example etilefrin, glyceryl trinitrate, isosorbide dinitrate and isosorbide mononitrate; antihypertensive agents, for example hydralazine; diuretics, for example furosemide and amiloride; beta-receptor blocking agents, for example propranolol and timolol; calcium channel blockers, for example amlodipine; ACE-inhibitors, for example kaptopril, lisinopril and fosinopril; serum lipid reducing agents, for example simvastatin; antipsoriatics, for example acitretin; antiasthmatic, for example terbutaline; antitussives, for example codeine and noscapine, antihistamines, for example clemastine, chlorpheniramine, cyproheptadine, loratadine, cetirizine and acrivastine; antidepressants and anti-sexual dysfunctions drugs, for example dapoxetine; anti-sexual dysfunction drugs, for example sildenafil (Viagra), tadalafil, vardenafil, cabergoline and pramipexole; antiepileptic, for example topiramate, antidepressants, for example amitriptyline and doxepin; oral health and anti-halitosis promoting agents, for example Lactobacillus reuteri and zinc; and mouth watering agents, for example malic acid.

The multi portion oral dosage form may also comprise chlorhexidine, nystatin, amphotericin, miconazole, phenylephrine, dextromethorphan, pseudoephedrine, acetaminophen, ibuprofen, ketoprofen, cannabidiol, delta-9-tetrahydrocannabinol, loperamide, famotidine, calcium carbonate, simethicone, pseudoephedrine, chlorpheniramine, methocarbomal, chlophedianol, ascorbic acid, menthol, thymol, methyl salicylate and eucalyptol, pectin, dyclonine, benzocaine, loratadine, terbutaline, propranolol, nitroglycerine and pharmaceutically acceptable salts and derivatives thereof.

In addition, the embodiments may also comprise herbal extracts such as, but not limited to, extracts from for example Echinacea (Echinacea augustifolia), Mastic gum (Pestacia lentiscus), Lavender (Lavandula augustifolia), Sage (Salvia officinalis) and isolated and/or synthesized pharmaceutically actives and their pharmaceutically acceptable salts, derivatives, complexes and prodrugs thereof.

The therapeutic area, if given, shall be regarded as a non-limiting example of a suitable therapeutic area for the stated pharmaceutically active agent(s), health promoting agent(s), salivation increasing agent(s) and herbal extract(s).

Further, the pharmaceutically active and/or health promoting agents and/or herbal extracts may be a smoking cessation compound(s) such as, but not limited to, nicotine and/or metabolites thereof, such as cotinine, nicotine N'-oxide, nornicotine, (S)-nicotine-N-β-glucuronide and mixtures, isomers, salts and complexes thereof in any form, varenicline, bupropion, nortriptyline, doxepin, fluoxetine, imipramine, moclobemide, conotoxinMII, epibatidine, A-85380, lobeline, anabasine, SIB-1508Y, SIB-1553A, ABT-418, ABT-594, ABT-894, TC-2403, TC-2559, RJR-2403, SSR180711, GTS-21, and/or cytisine and pharmaceutically acceptable salts, inclusion complexes, isomers, racemates, and prodrugs thereof.

In one embodiment the multi portion intra-oral dosage form may be used for delivering nicotine to a subject for treating e g tobacco dependence. The drug delivery system provides a potentially advantageous drug delivery system e g for a pharmaceutically active agent facilitating smoking cessation, where one portion may facilitate release of a pharmaceutically active agent in the saliva of the oral cavity thus providing oral health benefits and a second portion providing release of a smoking cessation agent such as nicotine for absorption into the systemic circulation of a subject. A number of nicotine replacement forms are available, but the present drug delivery system may provide new means for improving several features of a smoking cessation product such as increased compliance by adding conceptual aids to the subject, added health benefits to the subject, e g with respect to oral health as well as aid in reducing the initial nicotine craving as well as the craving over time and hence reducing the urge to use tobacco-containing material.

### Nicotine

With nicotine it is intended to include nicotine, 3-(1-methyl-2-pyrrolidinyl)-pyridine, with its base form, including synthetic nicotine as well as nicotine extracts from tobacco plants, or parts thereof, such as the genus Nicotiana alone or in combination; or pharmaceutically acceptable salts, inclusion complexes and prodrugs thereof.

In preferred embodiments, the nicotine in any form is selected from the group consisting of the free base form of nicotine, a nicotine salt, a nicotine derivative, such as a nicotine cation exchanger, a nicotine inclusion complex or nicotine in any non-covalent binding, nicotine bound to zeolites, nicotine bound to cellulose or starch micro spheres, and mixtures thereof.

Numerous nicotine salts are known, and may be used, e g the salts presented in Table 1, preferably monotartrate, hydrogen tartrate (also called bitartrate or bitartrate dihydrate), citrate, malate, and/or hydrochloride.

**Table 1. Examples of possible acids useful for nicotine salt formation**

| Acid | Molar ratio* of acid:nicotine |
|---|---|
| Formic | 2:1 |
| Acetic | 3:1 |
| Propionic | 3:1 |
| Butyric | 3:1 |
| 2-Methylbutyric | 3:1 |
| 3-Methylbutyric | 3:1 |
| Valeric | 3:1 |
| Lauric | 3:1 |
| Palmitic | 3:1 |
| Tartaric | 2:1 |
| Citric | 2:1 |
| Malic | 2:1 |
| Oxalic | 2:1 |
| Benzoic | 1:1 |
| Gentisic | 1:1 |
| Gallic | 1:1 |
| Phenylacetic | 3:1 |
| Salicylic | 1:1 |
| Phthalic | 1:1 |
| Picric | 2:1 |
| Sulfosalicylic | 1:1 |
| Tannic | 1:5 |
| Pectic | 1:3 |
| Alginic | 1:2 |
| Hydrochloric | 2:1 |
| Chloroplatinic | 1:1 |
| Silicotungstic | 1:1 |
| Pyruvic | 2:1 |
| Glutamic | 1:1 |
| Aspartic | 1:1 |

| | |
|---|---|
| * recommended at the time of production | |

The inclusion complex may include cyclodextrin complexation, such as complexation of the active pharmaceutically compound with cyclodextrin where preferably the cyclodextrin used is chosen among α-, β- and γ-cyclodextrin, the hydroxypropyl derivatives of α-, β- and γ-cyclodextrin, sulfoalkylether cyclodextrins such as sulfobutylether β-cyclodextrin, alkylated cyclodextrins such as the randomly methylated β-cyclodextrin, and various branched cyclodextrins such as glucosyl- and maltosyl-β-cyclodextrin.

Some suitable cation exchangers are given in below Table 2 and are further disclosed in U.S. 3,845,217. Preferred are nicotine cation exchangers of polyacrylates, such as the Amberlite collection from Rohm & Haas.

**Table 2 Examples of cation exchangers**

| Name | Type of crosslinked polymer | Manufacturer |
|---|---|---|
| Amberlite IRC 50 | Divinylbenzene-methacrylic acid | Rohm & Haas |
| Amberlite IRP 64 | Divinylbenzene-methacrylic acid | Rohm & Haas |
| Amberlite IRP 64M | Divinylbenzene-methacrylic acid | Rohm & Haas |
| BIO-REX 70 | Divinylbenzene-acrylic acid | BIO-RAD Lab. |
| Amberlite IR 118 | Styrene-divinylbenzene | Rohm & Haas |
| Amberlite IRP 69 | Styrene-divinylbenzene | Rohm & Haas |
| Amberlite IRP 69M | Styrene-divinylbenzene | Rohm & Haas |
| BIO-REX 40 | Phenolic | BIO-RAD Lab. |
| Amberlite IR 120 | Styrene-divinylbenzene | Rohm & Haas |
| Dowex 50 | Styrene-divinylbenzene | Dow Chemical |
| Dowex 50W | Styrene-divinylbenzene | Dow Chemical |
| Duolite C 25 | Styrene-divinylbenzene | Chemical Process Co |
| Lewatit S 100 | Styrene-divinylbenzene | Farbenfabriken Bayer |
| Ionac C 240 | Styrene-divinylbenzene | Ionac Chem. |
| Wofatit KP S 200 | Styrene-divinylbenzene | I.G. Farben Wolfen |
| Amberlyst 15 | Styrene-divinylbenzene | Rohm & Haas |
| Duolite C-3 | Phenolic | Chemical Process |
| Duolite C-10 | Phenolic | Chemical Process |
| Lewatit KS | Phenolic | Farbenfabriken Bayer. |
| Zerolit 215 | Phenolic | The Permutit Co. |
| Duolite ES-62 | Styrene-divinylbenzene | Chemical Process |
| BIO-REX 63 | Styrene-divinylbenzene | BIO-RAD Lab. |
| Duolite ES-63 | Styrene-divinylbenzene | Chemical Process |
| Duolite ES-65 | Phenolic | Chemical Process |
| Ohelex 100 | Styrene-divinylbenzene | BIO-RAD Lab. |
| Dow Chelating Resin A-1 | Styrene-divinylbenzene | Dow Chemical Company |
| CM Sephadex C-25 | Dextran | Pharmacia Fine Chemicals |
| SE Sephadex C-25 | Dextran | Pharmacia Fine Chemicals |
| Viscarin GP-109NF | Lambda-carrageenan | FMC Biopolymer |

### Amount and distribution of the nicotine in the oral formulation

The nicotine in any form according to the invention is formulated to provide the subject with a dose to achieve an effect. The effect may be to provide a sense of smoking satisfaction without smoking. Another effect of the administered nicotine in any form may be a reduction of the urge to smoke or use tobacco.

The effect may also be a combination of reduction of said urge and providing a sense of smoking satisfaction without smoking. The amount of the nicotine should be sufficient to provide such an effect in a subject. This amount may, of course, vary from person to person.

According to the invention, embodiments of the oral formulation comprise embodiments wherein nicotine in any form is present in an amount of 0.05 - 12 mg calculated as the free base form of nicotine per unit dose of the oral formulation. This may in different embodiments include 0.05, 0.1, 0.5, 1, 2, 3, 4, 5, 6, 7, 8, 9, 10, 11 or 12 mg calculated as the free base form of nicotine per unit dose, preferably in an amount of 0.1 - 6 mg, more preferably in an amount of 1 - 6 mg, and most preferably in an amount of 2 - 5 mg calculated as the free base form of nicotine per unit dose. Unit doses from 0.5 mg and upwards are normally considered to have a pharmaceutical effect.

The nicotine may be distributed in the present dosage forms in different embodiments. Different distributions of the nicotine throughout the present dosage forms will imply administration of the nicotine to the subject in different ways. This may, then, provide several possibilities to adjust the composition of the present dosage forms according to different needs of different subjects depending on the urge to smoke or use tobacco of the subject. In the below Examples are disclosed different such embodiments.

### Buffering agents

The portion(s) may also comprise a suitable system of buffering agent/s to facilitate nicotine administration. Absorption of nicotine from the oral cavity to the systemic circulation is dependent on the pH of the saliva, pH of the blood plasma and the pKa of nicotine, which is about 7.8. Thus, the level and type of buffering agent/s or combination thereof will affect the pH of the saliva and hence the absorption of nicotine in a free base form, which is the form predominantly absorbed through the mucosa. The buffering is designed so as to achieve a transient buffering of the saliva of a subject during melting, dissolution or dissolution of the oral formulation. As the change is transient, the pH will return to its normal value after a certain period of time.

In one embodiment, the multi portion intra-oral dosage form further comprises a buffer and/or a pH-adjusting agent, which upon administration to a subject transiently changes the pH of the saliva of the subject by 0.1 - 4 pH units, preferably by 0.2-3.5 pH units, most preferably by 0.5-3.0.

The buffering agent may be but are not limited to buffering agents from the group consisting of carbonate (including bicarbonate or sesquicarbonate), trometamol (2-amino-2-hydroxymethyl-1,3-propanediol, and also referred to as tromethamine, tris(hydroxymethyl)aminomethane and TRIS), glycinate, different phosphate systems such as trisodium phosphate, disodium hydrogen phosphate; and tripotassium phosphate, dipotassium hydrogen phosphate, glycerophosphate or citrate of an alkali metal (such as potassium or sodium, or ammonium), e g trisodium and tripotassium citrate, different hydroxides, amino acids, e g as per below Table 3, and mixtures thereof.

**Table 3 Examples of useful amino acids.**

| **Compound** | **CAS number** | **pKa value (in interval 8 - 9,6)** | **Solubility in water, g/kg** |
|---|---|---|---|
| Arginine | 74-79-3 | 9,00 | 182,6 ^{a)} |
| Aspargine | 70-47-3 | 8,73 | 25,1 |
| Glutamic acid | 56-86-0 | 9,58 | 8,61 ^{a)b)} |
| Glutamine | 56-85-9 | 9,00 | 42 |
| Glycine | 56-40-6 | 9,58 | 250,9 |
| Histidine | 71-00-1 | 9,09 | 43,5 |
| Isoleucine | 73-32-5 | 9,60 | 34,2 |
| Leucine | 61-90-5 | 9,58 | 22,0 |
| Lysine | 56-97-1 | 9,16 | Very soluble ^{a)b)} |
| Methionine | 63-68-3 | 9,08 | 56 |
| Phenylalanine | 63-91-2 | 9,09 | 27,9 |
| Serine | 56-45-1 | 9,05 | 50,2 |
| Threonine | 72-19-5 | 8,96 | 98,1 |
| Valine | 72-18-4 | 9,52 | 88,5 |
| Cysteic acid | 13100-82-8 | 8,70 | Very soluble |
| N-Glycylglycine | 556-50-3 | 8,10 | No information |
| Ornithine | 70-26-8 | 8,78 | Very soluble |

| | | | |
|---|---|---|---|
| a) reported as buffer in non-nicotine-containing pharmaceutical formulations. b) low or uncertain value on solubility in water. | | | |

The captioned data on the amino acids are taken from "Handbook of Chemistry and Physics", 85th edition; Table 7-1 ("20 standard amino acids that are the basic constituents of proteins") and Table 7-2 ("Amino acids and related compounds of biochemical importance").

### Other additives to the oral formulation

Other additives may be added optionally to the oral formulation. Optional additives comprise at least one or more additives selected from the group consisting of solvents, such as ethanol and water; co-solvents, such as propylene glycol; stabilisers, such as preservatives, e g antioxidants; softeners, such as sorbitol and glycerine; thickening/flowability agents, such as colloidal silicon dioxide; binding agents, such as xanthan gum; filling agents, such as mannitol, isomalt, cocoa powder and Crospovidone; solubilizers, such as Polysorbat 80 and Atmos 300; rubbers, lipid barriers, such as sucrose fatty acid esters and hydrogenated vegetable oils; film forming agents, such as gelatine, Pullulan, carrageenan, pectin, locust bean gum and xanthan gum; emulsifiers, such as pectin, soy lecithin, glycerol monostearate, castor oil and poloxamer; glidants, such as colloidal silicon dioxide; lubricants, such as magnesium stearate; coating agents, such as castor oil and sorbitol; melting vehicles, such as vegetable oils; sweeteners, flavors, aromatics, cooling agents, enhancers, colouring agents, vitamins, minerals, fluorine, breath fresheners, tooth whitening agents and mixtures thereof. According to the invention, at least one of such additives is optionally added to the product.

Enhancers may be added essentially to increase the transmucosal uptake of nicotine from the oral cavity.

Sweeteners are added essentially to improve the taste. Sweeteners comprise one or more synthetic or natural sugars, i e any form of carbohydrates suitable for use as sweetener, as well as so called artificial sweeteners such as saccharin, sodium saccharin, aspartame, e g NutraSweet^{®} acesulfame or Acesulfame K, potassium acesulfame, thaumatin, glycyrrhizin, sucralose, dihydrochalcone, miraculin, monellin, stevside, neotame, N-substituted APM derivatives, cyclamic acid and its salts and alitame.

Suitable sweeteners may be selected from the group consisting of sugar alcohols, such as sorbitol, xylitol, single sugars including sugars extracted from sugar cane and sugar beet (sucrose), dextrose (also called glucose), fructose (also called leavulose), and lactose (also called milk sugar); sorbitol, mannitol, glycerol, xylitol, erythritol, maltitol syrup (or hydrogenated starch hydrolyzate), isomalt, lactitol; and mixtures of sugars including glucose syrup, e g starch hydrolysates, containing a mixture of dextrose, maltose and a range of complex sugars, invert sugar syrup, e g sucrose inverted by invertase (also called sucrase or sacchrase) containing a mixture of dextrose and fructose, high sugar content syrups such as treacle and honey containing a mixture of particular leavulose, dextrose, maltose, lactitole, sucrose, resins, dextrin and higher sugars; and malt or malt extracts.

The flavor and aroma additives may comprise one or more synthetic or natural taste-masking, flavoring or aromatizing agents and may be added as liquids and/or as powder. Flavor and aroma agents may be selected from essential oils including distillations, solvent extractions, or cold expressions of chopped flowers, leaves, peel or pulped whole fruit comprising mixtures of alcohols, esters, aldehydes and lactones; essences including either diluted solutions of essential oils, or mixtures of synthetic chemicals blended to match the natural flavor of the fruit, e g strawberry, raspberry and black currant; artificial and natural flavors of brews and liquors, e g cognac, whisky, rum, gin, sherry, port, and wine; tobacco, coffee, tea, cocoa, and mint; fruit juices including expelled juice from washed, scrubbed fruits such as lemon, orange, and lime; spear mint, pepper mint, wintergreen, cinnamon, cacoe/cocoa, vanilla, liquorice, menthol, eucalyptus, aniseeds, nuts (e g peanuts, coconuts, hazelnuts, chestnuts, walnuts, colanuts), almonds, raisins; and powder, flour, or vegetable material parts including tobacco plant parts, e g genus Nicotiana, in amounts not contributing significantly to the level of nicotine, and ginger.

Colouring additives may be selected from dyes being approved as a food additive.

Stabilizing additives may be selected from the group consisting of antioxidants including vitamin E, i e tocopherole, ascorbic acid, sodium pyrosulfite, butylhydroxytoluene, butylated hydroxyanisole, edetic acid and edetate salts ; and preservatives including citric acid, tartaric acid, lactic acid, malic acid, acetic acid, benzoic acid, and sorbic acid. Preferred embodiments comprise an antioxidant as the stabiliser, and even more preferably the antioxidant vitamin E and/or butylated hydroxytoluene (BHT).

### Compressible Excipients

In one embodiment, at least one rapidly dissolving tablet portion includes one or more compressible excipients. In one embodiment the at least one rapidly dissolving intra-oral tablet portion comprises at least 40% by weight of such compressible excipients. In certain embodiments, the compressible excipient is in the form of a hydrate, and may be selected from organic compounds such as dextrose monohydrate, maltodextrin, lactose monohydrate, and dextrin, as well as inorganic compounds including dibasic calcium phosphate dihydrate, dibasic sodium phosphate dihydrate, dibasic sodium phosphate heptahydrate, dibasic sodium phosphate dodecahydrate, monobasic sodium phosphate monohydrate and monobasic sodium phosphate dihydrate. In one embodiment, the rapidly dissolving tablet portion includes a compressible excipient selected from the group consisting of isomalt, dextrose monohydrate, hydrogenated starch hydrolysate base, maltodextrin, lactose monohydrate, dextrin, mannitol, lactitol, sorbitol, xylitol, erythritol, sucrose, and lactose.

In one embodiment, the compressible excipient(s) are in the form of particles having an average particle diameter of from about 5 to about 1500 microns, more preferably with a particle size of 20 to about 1000 micron and most preferably with a particle size of 40 to 600 microns.

In one embodiment, a rapidly dissolving portion includes from about 5 to about 90 percent, such as from about 15 to about 75 percent, by weight of one or more compressible excipients. In one embodiment, the rapidly dissolving tablet portion includes at least 40 percent by weight of the one or more compressible excipients, based on the total weight of the disintegrative tablet portion.

### Water-Swellable Excipients

In one embodiment, the rapidly dissolving tablet portion further includes one or more water-swellable excipients. The water-swellable excipient may be selected from superdisintegrants such as crospovidone, croscarmellose, sodium starch glycolate, cellulose compounds such as microcrystalline cellulose, starches, alginic acid and inorganic clays such as bentonite, attapulgite, and magnesium aluminum silicate. In one embodiment, the water-swellable excipient is at least partially hydrated and selected from the group consisting of sodium starch glycolate, crospovidone, croscamellose, microcrystalline cellulose, starches, hydroxypropyl cellulose, and alginic acid.

In one embodiment, the amount of water-swellable excipient(s) in the rapidly dissolving tablet portion is from about 0.1 to about 5 percent by weight, such as from about 0.5 to about 3 percent by weight of the total weight of the rapidly dissolving tablet portion.

In one embodiment, the compressible excipient(s) is present in a greater amount than the water-swellable excipient(s). In one embodiment, the ratio of compressible excipient(s) to water-swellable excipient(s) in the disintegrative tablet portion is from about 1:1 to about 500:1 and more preferably 5:1 to about 200:1, and most preferably 10:1 to about 100:1.

### Effervescent Couple

In one embodiment, a disintegrative tablet portion includes one or more effervescent couples. In one specific embodiment, the effervescent couple includes one member from the group consisting of sodium bicarbonate, potassium bicarbonate, calcium carbonate, magnesium carbonate, sodium carbonate and one member selected from the group consisting of citric acid, malic acid, fumaric acid, tartaric acid, phosphoric acid, alginic acid.

In one embodiment, the combined amount of the effervescent couple(s) in the disintegrative tablet portion is from about 0.1 to about 20 percent by weight, such as from about 2 to about 10 percent by weight of the total weight of the disintegrative tablet portion.

### Additional information on ingredients

A rapidly dissolving tablet portion may include conventional ingredients, including other fillers, which include water-soluble compressible carbohydrates such as dextrose, sucrose, mannitol, sorbitol, maltitol, xylitol, lactose, and mixtures thereof; other conventional dry binders like polyvinyl pyrrolidone and the like; sweeteners such as aspartame, acesulfame potassium, sucralose, and saccharin; lubricants, such as magnesium stearate, stearic acid, talc, and waxes; preservatives; flavors; disintegrants, antioxidants; acidulants, such as but not limited to citric acid, malic acid, tartaric acid, ascorbic acid, and fumaric acid; surfactants; and coloring agents

A slowly dissolving portion or portions may comprise an excipient selected from, but not limited to, the group consisting of isomalt, sucrose, dextrose, dextrose monohydrate, corn syrup, lactitol, lycasin, mannitol, sorbitol, erythritol, xylitol, starches, gelatinized starches, maltodextrin, lactose, lactose monohydrate, dextrin, and mixtures and/or derivatives thereof. The slowly dissolving portion/s may comprise an excipient selected from but not limited to the group consisting of isomalt, sucrose, dextrose, corn syrup, lactitol, and lycasin, and mixtures and/or derivatives thereof.

Especially the rapidly dissolving portion/s may comprise an effervescent couple comprising e g one member selected from the group consisting of sodium bicarbonate, potassium bicarbonate, calcium carbonate, magnesium carbonate, and sodium carbonate and one member selected from the group consisting of citric acid, malic acid, fumaric acid, tartaric acid, and alginic acid.

The multi portion intra-oral dosage form may contain at least two portions which are rapidly dissolving. These at least two rapidly dissolving portions may at least partly cover the at least one slowly dissolving portion.

In one embodiment, the slowly dissolving portion(s) may cover(s) at least 20% of the surface of the rapidly dissolving portion.

### Further embodiments

Many embodiments are envisagable within the concept of the present invention. The following ones are examples on such. Also embodiments not mentioned below should be regarded as being included in the inventive concept.

The dosage form provides for including non-compatible ingredients, such as flavor components, buffers and pharmaceutically active or health promoting agents that are not compatible, by formulating such ingredients in separate portions.

In one embodiment, each portion of the multi portion intra-oral dosage form according to the invention comprises at least one item selected from a pharmaceutically active agent, a pH-buffering component, a pH-regulating component, a flavor, a barrier component, a color component, an adhesive component, a taste masking agent, a health promoting agent e g a tooth whitening agent, a breath freshening agent, an oral health promoting agent, and an anti-caries agent, preferably the pharmaceutically active or health promoting agent is selected from an agent for treating tobacco dependence, such as nicotine, and a nicotine mimicking agent. In one embodiment of the invention, it is preferred that the multi portion intra-oral dosage form is not a chewing gum or triturate.

A suitable embodiment relates to a multi portion intra-oral dosage form where at least one portion disintegrates more rapidly than at least one other portion, and where each portion comprises at least one item selected from a component for treating tobacco dependence, a pharmaceutically active component, a nicotine mimicking component, a pH-buffering component, a pH-regulating component, a flavor, a barrier component, a color component, an adhesive component, a taste masking agent, a tooth whitening agent, a breath freshening agent, an oral health promoting agent, and an anti-caries agent.

Where you have portions with different dissolution rates , the dissolution time for the slowest dissolving portion is at least two times longer than for the most rapidly dissolving portion, preferably 3 - 10 times longer, more preferably 3 - 5 times longer, than for the most rapidly dissolving portion. The rapidly dissolving portion(s) may comprise an effervescent component. At least two portions may be rapidly dissolving. The at least two rapidly dissolving portions may at least partly cover the at least one slowly dissolving portion, e g in such a way that the slowly dissolving portion(s) cover(s) at least 20% of the surface of the rapidly dissolving portion.

A multi portion intra-oral dosage form as above, wherein the rapidly dissolving portion comprises from about 5 to about 90 percent by weight of one or more of the compressible excipients based on the total weight of the disintegrative tablet portion, more preferably it comprrises from about 15 to about 75 percent and most preferably it includes at least 40 percent by weight of the one or more compressible excipients, selected from isomalt, dextrose monohydrate, maltodextrin, lactose monohydrate, dextrin, mannitol, lactitol, sorbitol, xylitol, erythritol, sucrose, or lactose, and mixtures thereof.

A multi portion intra-oral dosage form as above, wherein at least one rapidly dissolving portion comprises at least one pharmaceutically active agent selected from the group of phenylephrine, dextromethorphan, diphenhydramine, ambroxol, chlorpheniramine, cannabidiol, delta-9-tetrahydrocannabinol, chlophedianol, and pseudoephedrine, and wherein at least one slowly dissolving portion comprises at least one pharmaceutically active agent selected from the group of menthol, nicotine, dyclonine, pectin, benzocaine, thymol, methyl salicylate and eucalyptol.

A multi portion intra-oral dosage form according to the present invention, wherein the rapidly dissolving portion or portions has/have a hardness of less than about 15 kp/cm², and the slowly dissolving portion or portions has/have a hardness of greater than about 15 kp/cm².

A multi portion intra-oral dosage form according to the present invention, wherein the slowly dissolving portion or portions comprise(s) at least 50%, by weight, of a sugar selected from isomalt, sucrose, dextrose, corn syrup, lactitol, and lycasin, and mixtures and/or derivatives thereof. This embodiment may in a variant have the rapidly dissolving portion or portions further comprising an effervescent couple comprising a member selected from the group consisting of sodium bicarbonate, potassium bicarbonate, calcium carbonate, magnesium carbonate, and sodium carbonate and one member selected from the group consisting of citric acid, malic acid, fumaric acid, tartaric acid, and alginic acid.

A multi portion intra-oral dosage form according to the present invention , wherein the pharmaceutically active agent is in the form of particles that are further coated with a taste-masking polymer and wherein the average particle diameter of the particles is from about 50 microns to about 1000 microns.

A multi portion intra-oral dosage form according to the present invention, wherein at least one of the slowly dissolving portions comprises a plurality of openings exposing the surface area of the this/these portion/s, and substantially covers the surface area of at least one of the rapidly dissolving portions, whereby said slowly dissolving portion/s further comprise/s a plurality of indentations that, upon contact with the fluids in the oral cavity, are adapted to dissolve and expose the surface area of the rapidly dissolving portion/s.

A multi portion intra-oral dosage form as above, where at least one rapidly dissolving portion is substantially free from nicotine, wherein substantially free is less than 0.05 mg per unit dose.

A multi portion intra-oral dosage form as above, where at least one rapidly dissolving portion is a compressed portion and where at least one slowly dissolving portion has a matrix that is a hard candy glass.

A multi portion inter-oral dosage form as above, where the slowly or the rapidly dissolving portion(s) have indentation(s) and/or holes filled by the other dissolving portion(s). A multi portion inter-oral dosage form as above, where the dissolving portion(s) have indentation(s) and/or holes filled by the other dissolving portion(s).

A multi portion intra-oral dosage form according as above, wherein a pharmaceutically active agent within a rapidly dissolving portion is selected from the group consisting of chlorhexidine, L. reuteri, nystatin, amphotericin, miconazole, phenylephrine, dextromethorphan, pseudoephedrine, acetaminophen, ibuprofen, ketoprofen, loperamide, famotidine, calcium carbonate, simethicone, pseudoephedrine, chlorpheniramine, methocarbomal, chlophedianol, ascorbic acid, menthol, pectin, dyclonine, benzocaine, menthol, zinc acetate, sodium chlorite, amine fluoride, stannous fluoride, and pharmaceutically acceptable salts including also other pharmaceutically acceptable salts than those mentioned, derivatives including but not limiting to complexes thereof.

A multi portion intra-oral dosage form according to the present invention, wherein the face of at least one portion has a convex shape and the face of an adjoining portion has a concave shape.

A multi portion intra-oral dosage form according to the present invention having geometric similarities to a sphere, an open or closed oblong object, a sandwich, a hamburger or a torus.

A multi portion intra-oral dosage form according to the present invention having inter-portions layer/s comprising an edible adhesive-like material. Here the edible adhesive-like material may comprise an ingredient selected from the group consisting of polyethylene glycol, polyethylene oxide, polycaprolactone, carnauba wax, microcrystalline wax, oppanol, shellac wax and beeswax.

A formulation according to the present invention comprising nicotine in any form for use in therapy wherein the therapy is treatment of a disease selected from the group consisting of tobacco or nicotine dependence, Alzheimer's disease, Crohn's disease, Parkinson's disease, Tourette's syndrome, ulcerous colitis and post-smoking-cessation weight control.

A formulation according to the present invention comprising nicotine and/or metabolites thereof, such as cotinine, nicotine N'-oxide, nornicotine, (S)-nicotine-N-β-glucuronide and mixtures, isomers, salts and complexes thereof in any form for use in therapy wherein the therapy is treatment of a disease selected from the group consisting of tobacco or nicotine dependence, Alzheimer's disease, Crohn's disease, Parkinson's disease, Tourette's syndrome, ulcerous colitis and post-smoking-cessation weight control.

A multi portion intra-oral dosage form according to the invention, wherein at least one rapidly disintegrating portion comprises at least one pharmaceutically active agent selected from the group of phenylephrine, dextromethorphan, diphenhydramine, ambroxol, chlorpheniramine, cannabidiol, delta-9-tetrahydrocannabinol, chlophedianol, and pseudoephedrine, and wherein at least one slowly disintegrating portion comprises at least one pharmaceutically active agent selected from the group of menthol, nicotine in any form, dyclonine, pectin, benzocaine, thymol, methyl salicylate and eucalyptol.

In the multi portion intra-oral dosage form according to the invention one or more of the more rapidly disintegrating portion may substantially free from nicotine, wherein substantially free is less than 0.05 mg per unit dose.

A multi portion intra-oral dosage form according to the invention, **characterized in that** at least one portion comprises nicotine in any form and at least one other portion comprises Lactobacillus reuteri.

A multi portion intra-oral dosage form according to the invnetion, **characterized in that** at least one portion comprises nicotine in any form and at least one other portion comprises zinc where the zinc is in the form of zinc ions, zinc salt and/or zinc complex which may be physical or chemical.

A multi portion intra-oral dosage form according to any preceding claim, **characterized in that** at least one portion comprises terbutaline and at least one other portion comprises loratadine.

A multi portion intra-oral dosage form according to any preceding claim, **characterized in that** at least one portion comprises amitriptyline and at least one other portion comprises malic acid.

One may envisage a method for delivering nicotine or any other smoking cessation agent or nicotine-mimicking agent in any form to a subject comprising the steps of
a) administering to a subject a multi-portion intra-oral dosage form according to the present invention into the oral cavity of the subject, and
b) allowing the nicotine or any other smoking cessation agent or nicotine-mimicking agent in any form in the dosage form to be released in the saliva in the oral cavity and absorbed into the systemic circulation of the subject.

One may envisage a method for delivering nicotine or any other smoking cessation agent or nicotine-mimicking agent in any form to a subject comprising the captioned method and behavioural therapy.

A system for delivering nicotine or any other smoking cessation agent or nicotine-mimicking agent in any form to a subject, comprising a multi-portion intra-oral dosage form according to the present invention and at least one other means or method for obtaining reduction of the urge to smoke or use of tobacco.

A system for obtaining reduction of the urge to smoke or use of tobacco and/or for providing a sense of smoking satisfaction without smoking, comprising a multi-portion intra-oral dosage form according to the present invention and at least one other means or method for obtaining reduction of the urge to smoke or use of tobacco.

A system as stated above, wherein the at least one other means or method is a concomitant or concurrent means or method selected from the group consisting of administration through nasal sprays, transdermal patches, inhaling devices, lozenges, tablets, chewing gum and parenteral methods, subcutaneous methods, transmucosal methods, use of tobacco, and/or behavioural therapy. A variant of this system consists in the at least other means or method comprising administration of nicotine.

Use of a multi-portion intra-oral dosage form according to the present invention for obtaining a quick and/or sustained and/or complete reduction of the urge to smoke or use of tobacco and/or for providing a sense of smoking satisfaction without smoking and/or withdrawal symptom relief and/or for creating a nicotine kick.

### Examples

The skilled person may on the basis of the following examples envisage also other embodiments of the present invention. Batch sizes for the manufacture of the below formulations may be modified according to the actual need and to the actual production facilities.

### Example I

Preparation of a dual portion tablet, with differences in flavor, cooling intensity and hardness between portions, where a rapidly dissolving portion contains 0.5 mg nicotine (NRC) together with menthol flavor and a slowly dissolving portion contains 1.5 mg nicotine (NRC) with a lemon flavor. Thus the different flavors correlate to different fractions of the pharmaceutically active agent that in addition is released at different rates. The end of the dissolution of the rapidly disintigrating portion and the flavor of lemon indicates to the subject that roughly 25% of the pharmaceutically active agent has been released.

### Manufacturing method

The ingredients listed in below Table A1 and Table A2 are sieved and thereafter blended, each separately, according to methods known in the art e g using a double cone blender. The two portions of blended material are then compressed into tablets by means of direct compression. The powder compression may for example be performed using a double-sided rotary tablet press with individual filling stations and where each of the two portions, i e the rapidly dissolving tablet portion and the slowly dissolving portion, are subjected to pre-compression and main compression, respectively, to form a dual portion lozenge.

**Table A1: Components of the rapidly dissolving tablet portion.**

| Ingredients | Percent (w/w) | g/portion |
|---|---|---|
| Nicotine resin complex (20% nicotine) | 0.625 | 2.5* |
| Crospovidone | 0.75 | 3 |
| Microcrystalline Cellulose (Avicel PH 100) | 5 | 20 |
| Dextrose Monohydrate | 90.74 | 362.96 |
| Trometamol | 1.875 | 7.5 |
| Menthol | 0.25 | 1 |
| Coloring agent | 0.01 | 0.04 |
| Magnesium Stearate | 0.75 | 3 |
| TOTAL | 100.0 | 400 |

| | | |
|---|---|---|
| * Equivalent to 0.5 mg dose of nicotine base. | | |

**Table A2: Components of the slowly dissolving portion.**

| Ingredients | Percent (w/w) | mg/portion |
|---|---|---|
| Nicotine resin complex (20% nicotine) | 0.75 | 7.5* |
| Sorbitol | 95.75 | 957.5 |
| Trometamol | 1.0 | 10 |
| Sodium Carbonate | 0.5 | 5 |
| Lemon flavor | 1 | 10 |
| Magnesium Stearate | 1 | 10 |
| TOTAL | 100.0 | 1000.0 |

| | | |
|---|---|---|
| * Equivalent to 1.5 mg dose of nicotine base. | | |

### Example 2

Preparation of a dual portion tablet, with differences in texture and flavor between the portions, where one portion has a rough geometric pattern or form or shape and one portion has a smooth surface. The onset of a mint flavor provides the organoleptic sensation that indicates onset of the release of the pharmaceutically active agent to the subject, while a cinnamon flavor and rough surface texture of the corresponding portion is an indicator of a buffer that will facilitate the absorption of the pharmaceutically active agent. The tablet preparation also facilitates the inclusion of agents with potential compatibility issues.

### Manufacturing method

The same method as in *Example 1* is used. The upper punch though has a rough geometric pattern.

**Table B1: Components of the portion with a rough geometric pattern.**

| Ingredients | Percent (w/w) | mg per tablet |
|---|---|---|
| Isomalt | 97.49 | 389.96 |
| Cinnamon flavor | 1 | 4 |
| Sodium carbonate anhydrous | 0.5 | 2 |
| Coloring agent | 0.01 | 0.04 |
| Magnesium Stearate | 1 | 4 |
| TOTAL | 100.0 | 400 |

**Table B2: Components of the portion with a smooth geometric pattern.**

| Ingredients | Percent (w/w) | Mg/hard candy portion |
|---|---|---|
| Nicotine resin complex (20% nicotine) | 1.67 | 10* |
| Isomalt | 92.01 | 552.08 |
| L-Arginine | 4.32 | 25.92 |
| Mint | 1.00 | 6 |
| Magnesium Stearate | 1.00 | 6 |
| TOTAL | 100.00 | 600 |

| | | |
|---|---|---|
| * Equivalent to a 2.0 mg dose of nicotine base. | | |

### Example 3

Preparation of a dual portion tablet, with differences in texture between portions, where one portion is harder and has a rough geometric pattern and one portion is softer and has a smooth surface.

### Manufacturing method

The same method and the same formulation as in *Example 1* is used, The upper punch used though has a rough geometric pattern. Further, there is no added flavor. Hereby the difference in flakiness/crumbliness between the portions becomes more noticeable than in *Example 1.* The "flavor of the nicotine" in itself will act as a conceptual aid to the subject that the pharmaceutically active agent has started to be released and the fast dissolution of the flaky/crumbly and smooth surfaced portion will be an indicator to the subject that roughly 25 % of the pharmaceutically active agent has been released from the dosage form.

### Example 4

Preparation of a triple portion tablet, with differences in flavor and hardness between two rapidly dissolving portions and one slowly dissolving portion. The tablet comprises two rapidly dissolving portions, where one portion comprises 1 mg nicotine and the other portion comprises cinnamon flavor, and one slowly dissolving portion that comprises 3 mg nicotine. The subject's perception of a lemon flavor provides the organoleptic sensation indicating that roughly 75 % of the pharmaceutically active agent remains to be released. This preparation also facilitates the inclusion of agents in separate portions when agents have potential compatibility issues, e g cinnamon aldehyde and nicotine.

### Manufacturing method

Manufacturing principles according to the preceding examples are used.

**Table C1: Components of the first rapidly dissolving tablet portion containing 1.0 mg nicotine.**

| Ingredients | Percent (w/w) | mg/portion |
|---|---|---|
| Nicotine resin complex (20% nicotine) | 1.25 | 5* |
| Crospovidone | 0.75 | 3 |
| Microcrystalline Cellulose (Avicel PH100) | 5 | 20 |
| Dextrose Monohydrate | 91.345 | 360.46 |
| Trometamol | 1.875 | 7.5 |
| Menthol | 0.25 | 1 |
| Coloring agent | 0.01 | 0.04 |
| Magnesium Stearate | 0.75 | 3 |
| TOTAL | 100.0 | 400 |

| | | |
|---|---|---|
| * Equivalent to 1.0 mg dose of nicotine. | | |

**Table C2: Components of the second rapidly dissolving tablet portion containing cinnamon flavor.**

| Ingredients | Percent (w/w) | mg/portion |
|---|---|---|
| Crospovidone | 0.75 | 3 |
| Microcrystalline Cellulose (Avicel PH 100) | 5 | 20 |
| Dextrose Monohydrate | 93.22 | 367.96 |
| Cinnamon | 1.5 | 6 |
| Coloring agent | 0.01 | 0.04 |
| Magnesium Stearate | 0.75 | 3 |
| TOTAL | 100.0 | 400 |

**Table C3: Components of the slowly dissolving portion.**

| Ingredients | Percent (w/w) | mg/portion |
|---|---|---|
| Nicotine resin complex (20% nicotine) | 1.5 | 15* |
| Sorbitol | 96.5 | 965 |
| Trometamol | 1.0 | 10 |
| Sodium Carbonate | 0.5 | 5 |
| Lemon flavor | 1 | 10 |
| Magnesium Stearate | 1 | 10 |
| TOTAL | 100.0 | 1000.0 |

| | | |
|---|---|---|
| * Equivalent to 3.0 mg dose of nicotine base. | | |

### Example 5

Preparation of a double portion tablet as in *Example 1,* but where a pre-compressed slowly dissolving portion has the shape of a torus in which the powder of a rapidly dissolving portion is filled where after main compression is performed. The preparation provides organoleptic differences between the portions due to shape, mouth feel, flavor and hardness. The end of the dissolution of the rapidly disintigrating portion and the sensation of the flavor of lemon indicates to the subject that roughly 25% of the pharmaceutically active agent has been released.

### Example 6

Preparation of a double portion tablet with 2 mg nicotine containing a slowly dissolving boiled sugar portion and a rapidly dissolving tablet portion. The preparation provides organoleptic differences between the portions due to mouth feel, texture, hardness and flavor. The end of the dissolution of the rapidly disintigrating portion, the sensation of lemon-lime flavor and the change in mouth feel provides organoleptic sensations indicating to the subject that roughly 12% of the pharmaceutically active agent has been released.

### Manufacturing method

The method for preparing the slowly dissolving boiled sugar portion is as follows: Sieve the dry materials given in below Table D1. Add purified water, isomalt and maltitol solution to a stainless steel beaker. Mix and heat to ca 170° C during continuous mixing until the water is evaporated. Discontinue heating and cool to 135-140° C. Add nicotine bitartrate dihydrate and mix until fully dispersed. Add buffer components and mix at 120° C until dispersed. Thereafter add flavor and mix until uniform. While in its flowable state the hard candy portion blend is deposited into a circular stainless steel mold with dual flat faces. The resulting boiled sugar portion is allowed to cool and harden at room temperature for approximately15 minutes. The hard candy portion is then placed into a rubber mold. Approximately 30 milligrams of powdered polyethylene glycol (PEG) 3350 is evenly dispersed along one surface of the hard candy portion.

**Table D1: Components of the slowly dissolving boiled sugar portion blend.**

| Ingredients | Percent (w/w) | mg/hard candy portion |
|---|---|---|
| Nicotine bitartrate dihydrate (32.55% Nicotine) | 0.538 | 5.376* |
| Isomalt | 76.46 | 764.6 |
| Maltitol 75 % solution | 19.5 | 195 |
| Sodium carbonate anhydrous | 1 | 10 |
| Sodium Bicarbonate | 0.5 | 5 |
| Lemon-lime flavor | 2 | 20 |
| Purified water | - | - |
| TOTAL | 100.0 | 1000.0 |

| | | |
|---|---|---|
| *Equivalent to a 1.75 mg does of nicotine base | | |

A flat-faced compressed tablet is manufactured according to *Example 1* with components as per below Table D2.

**Table D2: Components of the rapidly dissolving tablet portion.**

| Ingredients | Percent (w/w) | mg per tablet |
|---|---|---|
| Nicotine bitartrate dihydrate (32.55% Nicotine) | 0.192 | 0.768* |
| Crospovidone | 0.75 | 3 |
| Microcrystalline Cellulose (Avicel PH 100) 100101) | 5 | 20 |
| Dextrose Monohydrate | 88.8 | 355.19 |
| Sodium carbonate anhydrous | 2.5 | 10 |
| Menthol-eucalypthus flavor | 2 | 8 |
| Coloring agent | 0.01 | 0.04 |
| Magnesium Stearate | 0.75 | 3 |
| TOTAL | 100.0 | 400 |

| | | |
|---|---|---|
| * Equivalent to a 0.25 mg dose of nicotine base. | | |

The rapidly dissolving tablet portion is adjoined to the boiled hard candy portion as follows: A flat-faced compressed tablet as per above is placed on top of the hard candy portion, and the resulting dosage form is placed into an oven providing a temperature being so high that the PEG 3350 melts and creates an adhesion between the compressed tablet and the hard candy portion. The resulting dual portion tablet is then allowed to cool at room temperature for 30 minutes and be removed from the rubber mold.

### Example 7

Preparation of a double portion tablet with 2 mg nicotine and 25 mg sildenafil citrate, containing a slowly dissolving hard boiled candy portion and a rapidly dissolving melt tablet portion. The preparation thus provides organoleptic discernability between the portions by means of hardness, texture, flavor and warming. The separation and dissolution of the melt tablet portion and the change of the aforementioned organoleptic sensations provide indicators to the subject that the pharmaceutically active agent included in that portion has just started to be released.

### Manufacturing method

The slowly dissolving hard boiled candy portion is prepared according to *Example 6 (D1).*

To prepare the melt tablet portion with composition as per below Table E1 a part of the hydrogenated soybean oil is first melted. Then the solid components, i e cocoa powder, mannitol, acesulfame-K, and the flavoring agents, if solid, are added and mixed. A reduction of particle size of the solid components is performed by milling in a roll-refiner. If the solid components have already got the required particle size, e g by milling before the mixing with the oil, roll refining is dispensed with. After treatment in the roll-refiner the mixture is mixed with the rest of the melted fatty components or remelted, if solidified, and mixed with the rest of the melted hydrogenated soybean oil. A mixing of the melt is performed in a suitable mixer. The liquid components, i e the soy lecithin and the flavoring agents (if liquid), are added at this stage.
The two portions, hard boiled candy and melt tablet, are then combined by dispensing the melt on top of the cooled and hardened hard boiled candy portion in a suitable mold. The melt is then allowed to solidify by cooling at 8-15° C for 2 hours. The complete dual portion dosage form is then broken from the mold and suitably packaged.

**Table E1: Components of the melt tablet portion.**

| Ingredients | Percent (w/w) | mg/melt tablet portion |
|---|---|---|
| Sildenafil citrate | 12.5 | 25.0 |
| Hydrogenated soybean oil | 40.0 | 80.0 |
| Cocoa powder | 32.05 | 64.1 |
| Mannitol | 13.35 | 26.7 |
| Acesulfame-K | 0.4 | 0.8 |
| Spicy flavour | 1.0 | 2.0 |
| Soy lecithin | 0.7 | 1.4 |
| TOTAL | 100.0 | 200.0 |

### Example 8

Preparation of seamless softgel concentric triple portion intra-oral capsules. The sensation of spicy-cinnamon flavor is an indicator that the pharmaceutically active agent soon will be released and the mint-menthol flavor indicates that the active is indeed being released and available to the subject for absorption and subsequently provides aid for abstaining from tobacco products.

**Table F1: Components of the triple portion capsules.**

| Ingredients | Percent in portion (w/w) | mg/capsule |
|---|---|---|
| *Ingredients in Centre Core Portion:* | | |
| | | |
| Nicotine free base | 2.2 | 2.0 |
| Medium chain triglycerides | 91.8 | 83.5 |
| Flavors (Mint and Menthol) and sweeteners | 5.5 | 5.0 |
| Colloidal silicon dioxide | 0.5 | 0.5 |
| | | |
| *Ingredients of Inner Shell Portion:* | | |
| | | |
| Sucrose fatty acid ester | 58.0 | 24.7 |
| Hydrogenated vegetable oil | 38.0 | 16.2 |
| Sodium carbonate anhydrous | 4.0 | 1.7 |
| | | |
| *Ingredients of Outer Shell Portion:* | | |
| | | |
| Gelatin | 77.0 | 6.5 |
| Sorbitol | 18.0 | 1.5 |
| Spicy Cinnamon | 3.0 | 0.3 |
| Sweeteners | 2.0 | 0.2 |
| Glycerin | | |
| *Weight Ratio:* | | |
| *Core*/*Inner shell*/*Outer shell* | 64/30/6 % | |
| *Total Capsule weight:* | | 142.1 mg |

### Manufacturing method

Seamless softgel capsules are manufactured by formation of droplets consisting of two or more concentric layers with ingredients as per above Table F1. The droplets are formed by feeding different liquids through concentric nozzles. The outermost nozzle feeds a hydrophilic solution consisting of gelatin and additives e g plasticizers. The one or more inner nozzles feed a lipophilic liquid, e g oils and triglycerids, wherein one or more active substances may be dispersed. The lipophilic centre and hydrophilic perimeter of the formed droplets ensure a good phase separation between shell and core contents. The formed capsules are then subjected to sequential processing steps such as cooling, drying, washing and selection of size and shape.

### Example 9

Preparation of a two portion cytisine mini-lozenge. The embodiment comprises cytisine and zinc in separate portions. The preparation provides a possibility for the subject to discern between the two portions by means of flavor, hardness and mouth feel.

**Table Q1: Components of a rapidly dissolving tablet portion.**

| Ingredients | Percent (w/w) | Mg/portion |
|---|---|---|
| Cytisine | 0.375 | 1.5 |
| Crospovidone | 0.75 | 3 |
| Microcrystalline Cellulose (Avicel PH100) | 5 | 20 |
| Dextrose Monohydrate | 90.99 | 362.96 |
| Trometamol | 1.875 | 7.5 |
| Mint-Menthol flavor | 0.25 | 1 |
| Coloring agent | 0.01 | 0.04 |
| Magnesium Stearate | 0.75 | 3 |
| TOTAL | 100 | 400 |

**Table Q2: Components of the slowly dissolving portion.**

| Ingredients | Percent (w/w) | mg/portion |
|---|---|---|
| Zinc Acetate dihydrate | 0.67 | 6.7* |
| Sorbitol | 95.83 | 958.3 |
| Trometamol | 1.0 | 10 |
| Sodium Carbonate | 0.5 | 5 |
| Lemon flavor | 1 | 10 |
| Magnesium Stearate | 1 | 10 |
| TOTAL | 100.0 | 1000.0 |

| | | |
|---|---|---|
| * Equivalent to 2.0 mg dose of zinc. | | |

### Manufacturing method.

The same method as in *Example 1* is principally used.

### Example 10

Preparation of a tablet containing 2mg nicotine and 10x10⁶ cfu Lactobacillus reuteri ATCC PTA-5289.

Preparation of a dual portion tablet where a rapidly dissolving portion contains Lactobacillus reuteri for improved oral health together with fruit flavor and a slowly dissolving portion contains 2.0 mg nicotine (NRC) with a mint flavor. Thus the differences in flavor and hardness between the portions provide an organoleptic sensation enabling the subject to discern between the releases of the different pharmaceutically active agents.

### Manufacturing method

The same method as in *Example 1* is used.

**Table H1: Components of the Lactobacillus reuteri containing rapidly dissolving tablet portion.**

| Ingredients | Mg or amount / portion |
|---|---|
| Lactobacillus reuteri ATCC PTA-5289 | 10x10⁶ cfu |
| Crospovidone | 3 |
| Microcrystalline Cellulose (Avicel PH100) 100101) | 20 |
| Dextrose Monohydrate | 371.96 |
| Fruit flavor | 1 |
| Coloring agent | 0.04 |
| Magnesium Stearate | 4 |

**Table H2: Components of the slowly dissolving nicotine containing portion.**

| Ingredients | Percent (w/w) | mg/portion |
|---|---|---|
| Nicotine resin complex (20% nicotine) | 1 | 10* |
| Sorbitol | 95.9 | 959 |
| Trometamol | 1.0 | 10 |
| Sodium Carbonate | 0.1 | 1 |
| Mint flavor | 1 | 10 |
| Magnesium Stearate | 1 | 10 |
| TOTAL | 100.0 | 1000.0 |

| | | |
|---|---|---|
| * Equivalent to a 2.0 mg dose of nicotine base. | | |

### Example 11

Preparation of a nicotine chewable tablet with a rapidly dissolving portion containing breath freshening agent: The preparation of the chewable portion includes wet granulation as one of the manufacturing steps (600 mg core weight). The zinc containing rapidly dissolving portion is discernable by mouth feel (removed from other portion by sucking on the dosage form) and cinnamon flavor. The onset of the nicotine is discernable to the subject by the mint flavor and mouth feel (the nicotine containing portion is intended to be chewed).

**Table I1: Components of the nicotine containing portion.**

| | 0 mg | 0,5 mg | 1 mg | 2 mg | 3 mg | 4 mg |
|---|---|---|---|---|---|---|
| | Unit | Unit | Unit | Unit | Unit | Unit |
| | formula | formula | formula | formula | formula | formula |
| **Active ingredients** | (mg) | (mg) | (mg) | (mg) | (mg) | (mg) |
| Nicotine hydrogen tartrate | 0 | 1,7 | 3,4 | 6,8 | 10,2 | 13,6 |
| | | | | | | |
| **Other ingredients** | | | | | | |
| Dextrose | 587 | 585 | 582 | 581 | 572 | 567 |
| PVP | 4 | 4 | 4 | 4 | 4 | 4 |
| PEG 6000 | 6 | 6 | 6 | 6 | 6 | 6 |
| Mint flavor | 3 | 3 | 3 | 3 | 3 | 3 |
| Water | q.s. | q.s. | q.s. | q.s. | q.s. | q.s. |

**Table I2: Components of the Zinc containing portion.**

| Ingredients | Percent (w/w) | mg/portion |
|---|---|---|
| Zinc Acetate dihydrate | 1.675 | 6.70 |
| Crospovidone | 0.75 | 3.00 |
| Microcrystalline Cellulose (Avicel PH100) 100101) | 5 | 20.00 |
| Dextrose Monohydrate | 88.315 | 353.26 |
| Sodium carbonate anhydrous | 2.5 | 10.00 |
| Cinnamon flavor | 1 | 4.00 |
| Coloring agent | 0.01 | 0.04 |
| Magnesium Stearate | 0.75 | 3.00 |
| TOTAL | 100 | 400.00 |

| | | |
|---|---|---|
| * Equivalent to 2.0 mg dose of zinc. | | |

### Manufacturing method:

The material used in the chewable tablet is prepared as follows: Nicotine hydrogen tartrate and dextrose powders are dry-blended and then granulated with a solution of PVP in water in a fluid bed granulator. The granulated material is then sieved and dry-blended with PEG. The material in the rapidly dissolving portion is blended. Double portion tablets are then compressed for example according to a similar method as outlined in *Example 1.*

### Example 12

Preparation of a two portion nicotine lozenge with breath freshening constituents. The embodiment comprises nicotine and the essential constituents of the commercially available mouth wash Listerine® marketed by McNeil. These constituents are mainly thymol, methyl salicylate, eucalyptol and menthol. The preparation provides a possibility for the subject to discern between the two portions by means of flavor and hardness.

**Table J1 Components of the breath freshening containing portion**

| Ingredients | Percent (w/w) | Mg/portion |
|---|---|---|
| Thymol, methyl salicylate, eucalyptol, and menthol * | 20.00 | 75 |
| Crospovidone | 2.67 | 10 |
| Mannitol | 75.86 | 283.29 |
| Menthol | 0.27 | 1 |
| Sweetener | 0.40 | 1.5 |
| Aerosil 200 | 0.5 | 1.875 |
| Coloring agent | 0.01 | 0.04 |
| Magnesium Stearate | 1.00 | 3.75 |
| Total | 100.00 | 375 |

| | | |
|---|---|---|
| * Spray dried material. | | |

**Table J2: Components of the nicotine containing portion.**

| Ingredients | Percent (w/w) | mg/portion |
|---|---|---|
| Nicotine resin complex (20% nicotine) | 1.5 | 15* |
| Sorbitol | 96.5 | 965 |
| Trometamol | 1.0 | 10 |
| Sodium Carbonate | 0.5 | 5 |
| Cinnamon flavor | 1 | 10 |
| Magnesium Stearate | 1 | 10 |
| TOTAL | 100.0 | 1000.0 |

| | | |
|---|---|---|
| * Equivalent to 3.0 mg dose of nicotine base. | | |

### Manufacturing method:

The same method as in *Example 1* is principally used.

### Example 13

Preparation of a two portion nicotine film with cinnamon. The embodiment comprises nicotine and the essential constituents of the mouthwash Listerine®, i e thymol, methyl salicylate, eucalyptol, and menthol. The preparation provides a possibility for the subject to discern between the two portions by means of flavor. The preparation follows basically the technical procedure of US 7,025,983 B2.

**Table K1. Composition of a Nicotine bi-layer film with 2 mg nicotine**

| Ingredient | mg per piece |
|---|---|
| *First portion* | |
| Ethanol | - |
| Klucel EF (HPC) | 33.856 |
| Nicotine BTDH | 6.144 |
| Sub-Total | 40.000 |
| *Second portion* | |
| Purified Water | 3.526 |
| Pullulan | 25.693 |
| TRIS Buffer | 27.648 |
| Polysorbate 80 | 0.74 |
| Cinnamon flavour | 2.388 |
| FDC Blue # 1 | 0.005 |
| Sub-Total | 60.000 |
| Total | 100.000 |

| | |
|---|---|
| *Nicotine /piece 2 mg measured as nicotine base. | |

### Manufacturing process:

1. In a beaker Nol dissolve Nicotine BTDH in ethanol.
2. Add Klucel and mix until hydrated.
3. To a beaker No 2 add Pullulan and FDC Blue #1 to 60 °C water. Mix to hydration.
4. To beaker No 2 add sucralose, mix to dissolve. Cool to room temperature.
5. To a beaker No3 add Polysorbate 80 and flavor, and mix. Transfer to beaker No 2. Mix until all is uniform.
6. Cast Pullulan solution from beaker No 2 onto substrate of desired thickness and dry with hot air.
7. Cast Klucel solution from beaker No 1 onto dried Pullulan layer of desired thickness and dry with hot air.
8. Cut into desired size. Size of e g 24 mm x 33 mm is appropriate.
Also multi-layer oral films are envisageable.

### Example 14

Preparation of a three portion gummies where the peppermint flavor indicates that the pharmaceutically active agent has started to be released.

**Table L1. Portion with 1 mg nicotine**

| **Ingredients** | **g per piece** |
|---|---|
| Isomalt | 1.85 |
| Sweetener | 0.5 |
| Water | 0.05 |
| Pectin | 0.05 |
| L-Arginine | 0.018 |
| Peppermint flavor | 0.05 |
| Nicotine bitartrate dihydrate | 0.0016 |
| Total | 2.5 g |

**Table L2. Separating portion**

| **Ingredients** | **g per piece** |
|---|---|
| Isomalt | 1.85 |
| Sweetener | 0.5 |
| Water | 0.05 |
| Pectin | 0.05 |
| L-Arginine | 0.018 |
| Menthol flavor | 0.05 |
| Nicotine bitartrate dihydrate | 0.0016 |
| Total | 2.5 g |

**Table L3. Cinnamon containing portion**

| **Ingredients** | **g per piece** |
|---|---|
| Isomalt | 1.85 |
| Sweetener | 0.5 |
| Water | 0.05 |
| Pectin | 0.05 |
| L-Arginine | 0.018 |
| Cinnamon flavor | 0.05 |
| Nicotine bitartrate dihydrate | 0.0016 |
| Total | 2.5 g |

### Manufacturing method:

1. Heat the isomalt to melting point and add sweetener and let the mixture cool.
2. To the cooled mixture, add pectin solution, L-Arginine and flavor.
3. Add nicotine bitartrate dihydrate, mix thoroughly.
4. Cast using starch moulds in desired shape and size using methods known in the art.
5. Prior to the solidification a second layer is added.

### Example 15

Preparation of a two portion lozenge with nicotine and zinc acetate. The change in flavor, cooling sensation, dissolution rate and mouth feel provide organoleptic sensations that indicate to the subject that the zinc acetate and nicotine are administered.

**Table M1: Components of the zinc containing portion.**

| Ingredients | Percent (w/w) | mg/portion |
|---|---|---|
| Zinc Acetate dihydrate | 1.34 | 6.7* |
| Sorbitol | 96.64 | 483.2 |
| Cinnamon flavor | 1 | 5 |
| Magnesium Stearate | 1 | 5 |
| Coloring agent | 0.02 | 0.1 |
| TOTAL | 100 | 500 |

| | | |
|---|---|---|
| * Equivalent to 2.0 mg dose of zinc. | | |

**Table M2: Components nicotine containing portion.**

| Ingredients | Percent (w/w) | mg/portion |
|---|---|---|
| Nicotine resin complex (20% nicotine) | 3 | 15 |
| Sorbitol | 93.5 | 467.5 |
| Trometamol | 1 | 5 |
| Sodium Carbonate | 0.5 | 2.5 |
| Menthol-Eucalyptus flavor | 1 | 5 |
| Magnesium Stearate | 1 | 5 |
| TOTAL | 100 | 500 |

| | | |
|---|---|---|
| * Equivalent to 3.0 mg dose of nicotine base. | | |

### Example 16

Preparation of a dual portion tablet where a rapidly dissolving portion contains terbutaline sulfate (5 mg) as a beta-adrenergic agonist bronchodilator together with menthol and a slowly dissolving portion contains Loratadine (10 mg) with a lemon flavor. The tablet provides a possibility for the subject to discern between the two pharmaceutically active agents by means of flavor and hardness.

**Table N1: Components of the Terbutaline containing tablet portion.**

| Ingredients | Percent (w/w) | Mg/portion |
|---|---|---|
| Terbutaline sulfate | 1.67 | 5 |
| Crospovidone | 3.33 | 10 |
| Mannitol | 93.15 | 279.48 |
| Menthol | 0.33 | 1 |
| Sweetener | 0.50 | 1.5 |
| Coloring agent | 0.01 | 0.03 |
| Magnesium Stearate | 1.00 | 3 |
| TOTAL | 100.00 | 300 |

**Table N2: Components of the Loratadine containing portion.**

| Ingredients | Percent (w/w) | mg/portion |
|---|---|---|
| Loratadin | 1 | 10 |
| Sorbitol | 96.65 | 966.5 |
| Citric acid | 0.35 | 3.5 |
| Lemon flavor | 1 | 10 |
| Magnesium Stearate | 1 | 10 |
| TOTAL | 100.0 | 1000.0 |

### Manufacturing method

The same method as in *Example 1* is principally used.

### Example 17.

Preparation of a dual portion tablet with Amitriptyline HCl 30 mg and 25 mg malic acid to provide mouth watering as a means to alleviate anticholinergic drug side effect within the same embodiment. The menthol-eucalyptus flavor and the mouth feel indicate to the subject that Amitriptyline is released and the onset of lemon flavor signals to the subject that the salivation aid is released.

**Table O1: Components of the Amitriptyline containing tablet portion.**

| Ingredients | Percent (w/w) | Mg/portion |
|---|---|---|
| Amitriptyline HCl | 7.5 | 30 |
| Crospovidone | 2.5 | 10 |
| Xylitol | 87.615 | 350.46 |
| Menthol -Eucalyptus flavor | 0.25 | 1 |
| Sweetner | 0.375 | 1.5 |
| Coloring agent | 0.01 | 0.04 |
| Aerosil 200 | 1 | 4 |
| Magnesium Stearate | 0.75 | 3 |
| TOTAL | 100 | 400 |

**Table O2: Components of the Malic acid containing portion.**

| Ingredients | Percent (w/w) | mg/portion |
|---|---|---|
| Malic Acid | 2.5 | 25 |
| Sodium Floride | 0.025 | 0.25 |
| Sorbitol | 951.25 | 947.25 |
| Citric acid | 0.35 | 3.5 |
| Lemon flavor | 1 | 10 |
| Aerosil 200 | 0.4 | 4 |
| Magnesium Stearate | 1 | 10 |
| TOTAL | 100.0 | 1000 |

### Manufacturing method.

The same method as in *Example 1* is used.

### Example 18.

Preparation of a two portion nicotine mini-lozenge. The embodiment comprises nicotine and cinnamon flavor in separate portions. The preparation provides a possibility for the subject to discern between the two portions by means of flavor, hardness and mouth feel.

**Table P1 Cinnamon containing portion**

| Ingredients | Percent (w/w) | Mg/portion |
|---|---|---|
| Mannitol | 94.42 | 75.54 |
| Crospovidone | 2.67 | 2.14 |
| Cinnamon | 1 | 0.80 |
| Sweetner | 0.4 | 0.32 |
| Aerosil 200 | 0.5 | 0.40 |
| Coloring agent | 0.01 | 0.01 |
| Magnesium Stearate | 1 | 0.80 |
| Total | 100 | 80.00 |

**Table P2: Nicotine containing portion.**

| Ingredients | Percent (w/w) | mg/portion |
|---|---|---|
| Nicotine bitartrate dihydrate | 2.85 | 2.85 |
| Sorbitol | 92.65 | 92.65 |
| Trometamol | 1 | 1 |
| Sodium Carbonate | 0.5 | 0.5 |
| Menthol-Eucalyptus flavor | 2 | 2 |
| Magnesium Stearate | 1 | 1 |
| TOTAL | 100 | 100 |

| | | |
|---|---|---|
| * Equivalent to 1.0 mg dose of nicotine. | | |

### Manufacturing method.

The same method as in *Example 1* is principally used.

### Example 19.

Preparation of a dual portion tablet with 2 mg nicotine and carbidopa 25 mg with 100 mg Levodopa. The portion that contains nicotine is mint flavored and rapidly dissolving to provide buccal release and uptake of nicotine and the organoleptic sensation of fruit flavour of the portion provides a signal cue for swallowing that part of the tablet. This embodiment provides advantages over that disclosed in patent application US20080260825A1 since this application do not address the serious issue with the high first passage effect for nicotine.

**Table A1: Components of the rapidly dissolving tablet portion.**

| Ingredients | Percent (w/w) | mg/portion |
|---|---|---|
| Nicotine resin complex (20% nicotine) | 2.5 | 10* |
| Crospovidone | 0.75 | 3 |
| Microcrystalline Cellulose (Avicel PH100) | 5 | 20 |
| Dextrose Monohydrate | 88.865 | 355.46 |
| Trometamol | 1.875 | 7.5 |
| Mint-Menthol flavor | 0.25 | 1 |
| Coloring agent | 0.01 | 0.04 |
| Magnesium Stearate | 0.75 | 3 |
| TOTAL | 100 | 400 |

| | | |
|---|---|---|
| * Equivalent to 2 mg dose of nicotine base. | | |

**Table A2: Components of the slowly dissolving portion.**

| Ingredients | Percent (w/w) | mg/portion |
|---|---|---|
| Levodopa | 2.5 | 25 |
| Carbidopa | 10.0 | 100 |
| Sorbitol | 84.0 | 957.5 |
| Trometamol | 1.0 | 10 |
| Sodium Carbonate | 0.5 | 5 |
| Lemon flavor | 1.0 | 10 |
| Magnesium Stearate | 1.0 | 10 |
| TOTAL | 100 | 1000.0 |

### Manufacturing method.

The same method as in *Example 1* is principally used.

Also other embodiments than those presented in the captioned examples are envisageable by the present invention. For example you may manufacture a medicated chewing gum according to the present invention.

## Claims

1. A multi portion intra-oral dosage form comprising at least one pharmaceutically active agent or health promoting agent, wherein there is a discernable difference in organoleptic sensation between at least two portions, where preferably the organoleptic sensation/s is/are related to perception of flavor, effervescence, olfactory sensation, viscosity, elasticity, rheology, texture, mouth feel and difference in disintegration rate.

2. A multi portion intra-oral dosage form according to claim 1, where the portions have different disintegration rates.

3. A multi portion intra-oral dosage form according to claim 2, wherein at least one more rapidly disintegrating portion comprises a compressible excipient selected from isomalt, dextrose monohydrate, maltodextrin, lactose monohydrate, dextrin, mannitol, lactitol, sorbitol, xylitol, erythritol, sucrose, and lactose, and mixtures or derivatives thereof; wherein preferably the at least one more rapidly disintegrating portion comprises from about 5 to about 90 percent by weight of one or more of the compressible excipients based on the total weight of the disintegrating portion, more preferably it comprises from about 15 to about 75 percent and most preferably it includes at least 40 percent by weight of the one or more compressible excipients.

4. A multi portion intra-oral dosage form according to claim 3, where the compressible excipient is in the form of particles with an average particle diameter of about 5 to about 1500 microns more preferably with a particle size of 20 to about 1000 micron and most preferably with a particle size of 40 to 600 microns.

5. A multi portion intra-oral dosage form according to claim 3 or claim 4, wherein the at least one more rapidly disintegrating portion further comprises a water-swellable excipient selected from sodium starch glycolate, crospovidone, croscarmellose, microcrystalline cellulose, starches, hydroxypropyl cellulose, and alginic acid; wherein preferably the weight ratio of the compressible excipient to the water-swellable excipient is from about 1:1 to about 500:1 and more preferably 5:1 to about 500:1, and most preferably 10:1 to about 200:1.

6. A multi portion intra-oral dosage form according to claim 3 or claim 4, wherein the more rapidly disintegrating portion or portions further comprise(s) an effervescent couple comprising a member selected from the group consisting of sodium bicarbonate, potassium bicarbonate, calcium carbonate, magnesium carbonate, and sodium carbonate and one member selected from the group consisting of citric acid, malic acid, fumaric acid, tartaric acid, and alginic acid.

7. A multi portion intra-oral dosage form according to any one of claims 2 to 6 where the disintegration time for the slowest dissolving portion is at least two times longer than for the most rapidly disintegrating portion, preferably the disintegration time for the most slowly disintegrating portion is 3 - 10 times longer, more preferably 3 - 5 times longer, than for the most rapidly disintegrating portion.

8. A multi portion intra-oral dosage form according to any of claims 2 to 7 where at least one of the more rapidly disintegrating portion is a compressed portion and where at least one of the more slowly disintegrating portion has a matrix that is a hard candy glass.

9. A multi portion intra-oral dosage form according to any preceding claim, where each portion comprises at least one item selected from a component for treating tobacco dependence, a pharmaceutically active component, a nicotine mimicking component, a pH-buffering component, a pH-regulating component, a flavor, a barrier component, a color component, an adhesive component, a taste masking agent, a tooth whitening agent, a breath freshening agent, an oral health promoting agent, and an anti-caries agent.

10. A multi portion intra-oral dosage form according to claim 9, wherein at least one portion further comprises a buffer and/or a pH-adjusting agent, which upon administration to a subject transiently changes the pH of the saliva of the subject by 0.1 - 4 pH units, preferably by 0.2-3.5 pH units, most preferably by 0.5-3.0.

11. A multi portion intra-oral dosage form according to any preceding claim, being a lozenge, a tablet, an oral film, a chewing gum, a sublingual tablet, a troche, a lolly pop, a hard boiled candy, a chocolate lens, a micro bead, a wine gum, a semi solid, or a combination thereof.

12. A multi portion intra-oral dosage form according to any preceding claim wherein at least one of the portions comprises a component for treating tobacco dependence, which is nicotine in any form including a nicotine pro-drug and/or nicotine metabolite, preferably selected from the group consisting of a nicotine salt, the free base form of nicotine, a nicotine derivative, such as a nicotine cation exchanger, a nicotine inclusion complex or nicotine in any non-covalent binding, nicotine bound to zeolites, and nicotine bound to cellulose including microcrystalline cellulose, or starch micro-spheres; and cotinine, nicotine N'-oxide, nornicotine, (S)-nicotine-N-β-glucuronide or isomers thereof, and/or mixtures thereof; wherein the nicotine inclusion complex is preferably a cyclodextrin complex, where the cyclodextrin used is chosen among α-, β- and γ-cyclodextrin, hydroxypropyl derivatives of α-, β- and γ-cyclodextrin, sulfoalkylether cyclodextrins such as sulfobutylether β-cyclodextrin, alkylated cyclodextrins such as randomly methylated β-cyclodextrin, and branched cyclodextrins such as glucosyl- and maltosyl-β-cyclodextrin; and wherein the nicotine cation exchanger is preferably a polyacrylate cation exchanger; and wherein the nicotine salt is preferably monotartrate, hydrogen tartrate, citrate, malate and/or hydrochloride salt.

13. A multi portion intra-oral dosage form according to claim 12, wherein the nicotine compound is present in an amount of 0.05 - 12 mg, preferably in an amount of 0.1 - 6 mg, more preferably in an amount of 1 - 6 mg, and most preferably in an amount of 2 - 5 mg calculated as the free base form of nicotine per unit dose.

14. A multi portion intra-oral dosage form according to any preceding claim, comprising a component for treating tobacco dependence being selected from one or more of varenicline, bupropion, nortriptyline, doxepin, fluoxetine, imipramine, moclobemide, conotoxinMII, epibatidine, A-85380, lobeline, anabasine, SIB-1508Y, SIB-1553A, ABT-418, ABT-594, ABT-894, TC-2403, TC-2559, RJR-2403, SSR180711, GTS-21, and/or cytisine.

15. A multi portion intra-oral dosage form according to any preceding claim, wherein at least one of the portions comprises one or more pharmaceutically active agent(s) is/are chosen from the antiinflammatory agents diclofenac, ketorolac, indometacin, tornoxicam, piroxicam, tenoxicam, ketoprofen, celecoxib and roficoxib; the muscle relaxants orphenadrine and baclofen; the drugs affecting bone mineralization alendronic acid and risedronic acid; the analgesics propoxyphene, buprenorfin, ketobenidon, hydromorphone, tramadol, morphine, and tapentadol; the antimigraine preparations: dihydroergotamine, ergotamine, eletriptan, naratriptan, rizatriptan, sumatriptan and zolmitriptan; the anti-Parkinson drugs pramipexole, ropinirole, levodopamine, carbidopamine and selegiline; the anxiolytics alprazolam, diazepam, lorazepam and oxazepam; the hypnotics flunitrazepam, midazolam, nitrazepam, triazolam, zaleplone, zopiclone, zolpiderm, clometiazole and propiomazine; the psychostimulant caffeine; the drugs against substance dependence bupropione, lobeline, naltrexone and methadone; the gastric ulcer remedy famotidine; the antispasmodic hyoscyamine; the antiemetics metoclopramide, ondansetron, scopolamine, hyoscine, perfenazine, procloperazine and haloperidol; the antidiabetic agent rosiglitazone; the cardiovascular agents etilefrin, glyceryl trinitrate, isosorbide dinitrate and isosorbide mononitrate; the antihypertensive agent hydralazine; the diuretics furosemide and amiloride; the beta-receptor blocking agents propranolol and timolol; the calcium channel blocker amlodipine; the ACE-inhibitors kaptopril, lisinopril and fosinopril; the serum lipid reducing agent simvastatin; the antipsoriatic acitretin; the antiasthmatic terbutaline; the antitussives codeine and noscapine, and the antihistamines clemastine, chlorpheniramine, cyproheptadine, loratadine, cetirizine and acrivastine; the antidepressant and anti-sexual dysfunction drug dapoxetine; the anti-sexual dysfunction drugs sildenafil (Viagra), tadalafil, vardenafil, cabergoline and pramipexole; the antiepileptic topiramate; and the breath freshening agent and agent for treating Wilson's disease, zinc and the oral health promoting agent Lactobacillus reuteri.

16. A multi portion intra-oral dosage form according to any of claims 2 to 15, wherein at least one rapidly disintegrating portion comprises at least one pharmaceutically active agent selected from the group of phenylephrine, dextromethorphan, diphenhydramine, ambroxol, chlorpheniramine, cannabidiol, delta-9-tetrahydrocannabinol, chlophedianol, and pseudoephedrine, and wherein at least one slowly disintegrating portion comprises at least one pharmaceutically active agent selected from the group of menthol, nicotine in any form, dyclonine, pectin, benzocaine, thymol, methyl salicylate and eucalyptol.

17. A multi portion intra-oral dosage form according to any to any of claims 2 to 16 where at least one of the more rapidly disintegrating portion is substantially free from nicotine, wherein substantially free is less than 0.05 mg per unit dose.

18. A multi portion intra-oral dosage form according to any preceding claim, **characterized in that** at least one portion comprises nicotine in any form and at least one other portion comprises Lactobacillus reuteri.

19. A multi portion intra-oral dosage form according to any preceding claim, **characterized in that** at least one portion comprises nicotine in any form and at least one other portion comprises zinc where the zinc is in the form of zinc ions, zinc salt and/or zinc complex which may be physical or chemical.

20. A multi portion intra-oral dosage form according to any preceding claim, **characterized in that** at least one portion comprises terbutaline and at least one other portion comprises loratadine.

21. A multi portion intra-oral dosage form according to any preceding claim, **characterized in that** at least one portion comprises amitriptyline and at least one other portion comprises malic acid.

22. A multi portion intra-oral dosage form according to any preceding claim, **characterized in that** at least one portion comprises herbal extracts from Echinacea (Echinacea augustifolia), Mastic gum (Pestacia lentiscus), Lavender (Lavandula augustifolia), Sage (Salvia officinalis) and isolated and/or synthesized pharmaceutically actives and their pharmaceutically acceptable salts,derivatives, complexes and prodrugs thereof.

23. A multi portion intra-oral dosage form according to any preceding claim, **characterized in that** at least one portion comprises one or more sweeteners selected from saccharin, sodium saccharin, aspartame, e g NutraSweet@, acesulfame or Acesulfame K, potassium acesulfame, thaumatin, glycyrrhizin, sucralose, dihydrochalcone, alitame, miraculin, monellin, stevside, neotame, N-substituted APM derivatives, cyclamic acid and its salts, alitame, sorbitol, xylitol, single sugars including sugars extracted from sugar cane and sugar beet (sucrose), dextrose (also called glucose), fructose (also called leavulose), and lactose (also called milk sugar); sorbitol, mannitol, glycerol, xylitol, erythritol, maltitol syrup (or hydrogenated starch hydrolyzate), isomalt, lactitol; and mixtures of sugars including glucose syrup, e g starch hydrolysates, containing a mixture of dextrose, maltose and a range of complex sugars, invert sugar syrup, e g sucrose inverted by invertase (also called sucrase or sacchrase) containing a mixture of dextrose and fructose, high sugar content syrups such as treacle and honey containing a mixture of particular leavulose, dextrose, maltose, lactitole, sucrose, resins, dextrin and higher sugars; and malt or malt extracts.

24. Use of a multi-portion intra-oral dosage form according to any one of claims 1 to 19 and 22 to 23 comprising nicotine in any form for obtaining a quick and/or sustained and/or complete reduction of the urge to smoke or use of tobacco and/or for providing a sense of smoking satisfaction without smoking and/or withdrawal symptom relief and/or for creating a nicotine kick.

25. A multi-portion intra-oral dosage form according to any of claims 1 to 23 comprising nicotine in any form for use in therapy wherein the therapy is treatment of a disease selected from the group consisting of tobacco or nicotine dependence, Alzheimer's disease, Crohn's disease, Parkinson's disease, Tourette's syndrome, ulcerous colitis and post-smoking-cessation weight control.
